# EUROPÄISCHE PATENTANMELDUNG

(11) **EP 1 002 500 A1**
(43) Veröffentlichungstag der Anmeldung: **24.05.2000**
(21) Anmeldenummer: 99120710.1
(22) Anmeldetag: 19.10.1999
(51) Int. Cl.: A61B 17/34

(54) **Katheterset für die Plexusanästhesie**

(30) Priorität: 17.11.1998 DE 29820525 U
(71) Anmelder: B. BRAUN MELSUNGEN AG, 34212 Melsungen (DE)
(72) Erfinder: Pape-Werlich, Wolfgang, 37075 Göttingen (DE); Seeber, Manfred, 34587 Felsberg/Gensungen (DE); Sippel, Martin, Dr., 34212 Melsungen (DE)
(74) Vertreter: Selting, Günther, Dipl.-Ing.

(57) **Zusammenfassung**

Das Katheterset weist einen Kurzkatheter (10) auf, der auf eine Stahlkanüle (11) aufschiebbar ist. Mit der Stahlkanüle (11) wird der Kurzkatheter (10) in den Körper des Patienten eingeführt, wobei eine Nervenstimulation mit einem elektrischen Stimulationsgerät erfolgt, dessen Impulse auf die Kanülenspitze (17) gegeben werden. Nach Erreichen des Zielortes wird die Stahlkanüle (11) herausgezogen und in den Kurzkatheter (10) wird ein Plexuskatheter (30) mit darinliegendem Stimulationsdraht (31) eingeschoben. Der Stimulationsdraht (31) ist an seinem distalen Ende als Stimulationselektrode (32) ausgebildet und am proximalen Ende weist er einen elektrischen Anschlußbereich (33) auf, der mit einer Anschlußvorrichtung (35) für Stimulationsimpulse verbunden werden kann. Das weitere Vorschieben des Plexuskatheters (30) im Nervenbereich erfolgt ebenfalls unter kontrollierter Stimulation, so daß die für die Injektion eines Anästhetikums günstigste Position bestimmt werden kann.

## Beschreibung

Die Erfindung betrifft ein Katheterset für die Plexusanästhesie zur Durchführung einer kontinuierlichen Nervenblockade.

Aus DE 32 29 466 A1 ist eine Punktier- und Katheterisierungsvorrichtung bekannt, die eine Stahlkanüle und einen auf die Stahlkanüle aufschiebbaren Kurzkatheter aufweist. Die elektrisch leitende Stahlkanüle ist an ihrem rückwärtigen Ende mit einem elektrischen Anschlußteil ausgestattet, so daß ihre Spitze als Elektrode für die periphere Nervenstimulation benutzt werden kann. Bei der peripheren Nervenstimulation wird ein Stimulationsgerät benutzt, das elektrische Stimulationsimpulse erzeugt. Diese Impulse werden durch die Stahlkanüle auf das angrenzende Körpergewebe übertragen. Wenn sich die Stahlkanüle in entsprechendem Abstand zu dem zu blockierenden Nervenstrang befindet, reagiert dieser auf die Stimulationsimpulse. Anhand von Muskelkontraktion kann der Anästhesist feststellen, ob die Stahlkanüle das gewünschte Zielgebiet erreicht hat. Wenn dies der Fall ist, kann anschließend der Plexuskatheter verlegt werden, durch den das Anästhetikum zugeführt wird. Diese kontinuierliche Plexusanästhesie wird zur postoperativen Analgesie oder bei längeren Operationen angewandt.

Mit dem bekannten Katheterset wird unter Nervenstimulation das Zielgebiet bestimmt, bis zu der die Stahlkanüle vorgeschoben wird. Anschließend wird entweder durch einen Kurzkatheter hindurch oder über einen Stimulationsdraht der Plexuskatheter bis an dieselbe Stelle vorgeschoben. Dabei besteht allerdings auch die Möglichkeit, den Plexuskatheter entlang der Nerven noch weiter vorzuschieben. Während die Stahlkanüle nur so weit vorgeschoben wird, bis sie die Faszienwand durchstochen hat, kann es zweckmäßig sein, den Katheter über den von der Stahlkanüle erzeugten Einstichkanal hinaus weiter vorzuschieben, bis die Katheterspitze eine Stelle erreicht, die wegen des Zusammentreffens zahlreicher Nerven für die Plexusanästhesie besonders geeignet ist. Dieses Vorschieben des Plexuskatheters erfolgt im Stand der Technik blind, d.h. ohne Stimulationsüberwachung.

Der Erfindung liegt die Aufgabe zugrunde, ein Katheterset für die Plexusanästhesie zu schaffen, mit dem es möglich ist, einen Plexuskatheter hochgenau zu verlegen, um auf einfache Weise die für die kontinuierliche Plexusanästhesie wirksamste Stelle aufzusuchen.

Die Lösung dieser Aufgabe erfolgt erfindungsgemäß mit den im Patentanspruch 1, Patentanspruch 5 bzw. Patentanspruch 8 angegebenen Merkmalen.

Die erfindungsgemäßen Lösungen nach den Ansprüchen 1, 4 und 7 unterscheiden sich dadurch, daß der Anspruch 1 auf der Braunülen-Technik und der Anspruch 5 auf der Seldinger-Technik und der Anspruch 8 auf der Möllmann-Technik beruht. Bei der Braunülen-Technik wird mit Hilfe einer Stahlkanüle ein Kurzkatheter verlegt und anschließend wird der Plexuskatheter durch den Kurzkatheter hindurch vorgeschoben. Der Kurzkatheter wird dann über den Plexuskatheter abgezogen. Bei der Seldinger-Technik wird ebenfalls mit einer Stahlkanüle ein Kurzkatheter verlegt und durch diesen wird ein Stimulationsdraht vorgeschoben. Über den Stimulationsdraht wird dann der Plexuskatheter eingeführt.

Erfindungsgemäß ist bei allen Varianten ein Stimulationsdraht vorgesehen, der an seinem distalen Ende als Stimulationselektrode dient und am proximalen Ende die erforderliche elektrische Anschlußmöglichkeit aufweist. Bei der Braunülen-Technik wird der Stimulationsdraht zusammen mit dem Plexuskatheter eingeführt, wobei der Stimulationsdraht auch Bestandteil des Plexuskatheters sein kann. Dadurch ist es möglich, das Vorschieben des Plexuskatheters über die Punktionstiefe hinaus unter Nervenstimulation vorzunehmen, indem an den Stimulationsdraht Stimulationsimpulse gelegt werden. Dies erlaubt eine Feinpositionierung der Katheterspitze unter Nervenstimulation. Damit ist es möglich, den Katheter auf einfache Weise mit hoher Genauigkeit in demjenigen Bereich zu plazieren, in dem das durch den Katheter zu applizierende Medikament die größte Wirksamkeit entfaltet.

Bei der Braunülen-Version, bei der der Plexuskatheter durch den Kurzkatheter hindurchgeschoben wird, kann der Stimulationsdraht als elektrisch leitendes Element integraler Bestandteil des Plexuskatheters sein. Der Stimulationsdraht steht geringfügig hinter der geschlossenen Spitze des Plexuskatheters zurück. Kurz hinter dem distalen Ende des Plexuskatheters sind seitliche Öffnungen angebracht, die als Emissionsorte für die Stromlinien dienen. Hierbei ist hervorzuheben, daß die seitliche Abgabe der Stromlinien für die Stimulation effizienter ist, da der Katheter parallel zum Nerv vorgeschoben wird. Am proximalen Ende ist er mit einem Steckverbinder oder Anschlußbereich versehen. Die geschlossene distale Katheterspitze ermöglicht eine präzise Positionierung des Stimulationsdrahtes im Plexuskatheter. Zum anderen werden Gefahren durch einen aus dem Plexuskatheter vorstehenden Draht vermieden. Ein weiterer Vorteil besteht darin, daß durch das Loch bzw. die Löcher im Plexuskatheter die Größe der Stimulationselektrode exakt definiert wird. Schließlich kann der Stimulationsdraht verhältnismäßig steif ausgeführt werden, so daß eine hohe Katheterstabilität und ein knickfreier Vorschub gewährleistet sind.

Die dritte Variante der Erfindung betrifft ein Katheterset nach DE 44 43 672 C2 (Möllmann et al.), jedoch mit in dem Plexuskatheter verlaufendem oder integriertem Stimulationsdraht.

Im folgenden werden unter. Bezugnahme auf die Zeichnungen Ausführungsbeispiele der Erfindung näher erläutert.

Es zeigen:
- Fig. 1: eine erste Ausführungsform des Kathetersets nach dem Braunülen-Prinzip zusammen mit einem Stimulationsgerät, das bei der Verlegung des Plexuskatheters benutzt wird,
- Fig. 2: eine vergrößerte Darstellung der Einzelheit II von Fig. 1,
- Fig. 3: ein Katheterset nach dem Prinzip der Seldinger-Technik und
- Fig. 4: ein Ausführungsbeispiel nach der Möllmann-Technik.

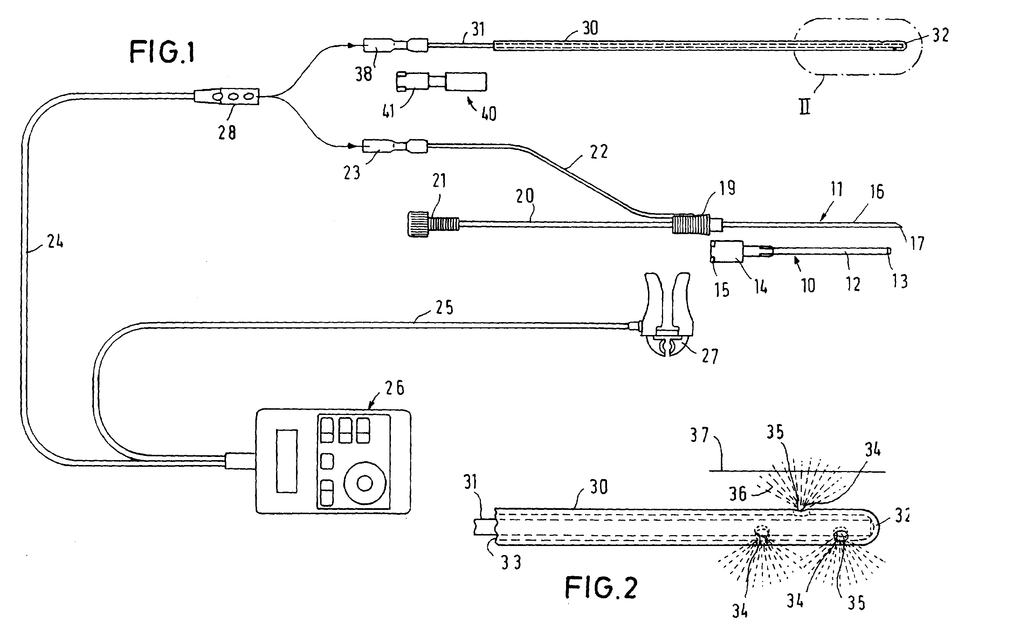

Das Katheterset nach Fig. 1 weist einen Kurzkatheter 10 und eine Stahlkanüle 11 auf, wobei der Kurzkatheter 10 über die Stahlkanüle 11 geschoben werden kann. Der Kurzkatheter 10 ist nach Art einer Braunüle ausgebildet. Er besitzt ein halbstarres Rohr 12 aus Kunststoff, wobei die Katheterspitze 13 kegelstumpfförmig verjüngt ist. Am distalen Ende des Rohres 12 ist ein Katheteransatz 14 aus starrem Kunststoff befestigt, welcher einen (nicht dargestellten) Innenkonus und Luer-Lock-Verbindungselemente 15 zum Anschließen einer Spritze oder eines Schlauchkonnektors aufweist.

Die Stahlkanüle 11 besteht aus einem Kanülenrohr 16 aus Stahl, das am distalen Ende eine asymmetrische scharfe Spitze 17 aufweist. Das Kanülenrohr 16 kann mit einer isolierenden Beschichtung aus Kunststoff umhüllt sein, die nur die blanke Spitze 17 freiläßt. Am proximalen Ende des Kanülenrohrs 16 befindet sich ein Kanülenansatz 19 aus Kunststoff/Metall. Aus diesem Kanülenansatz 19 ragt ein flexibler Schlauch 20 heraus, der mit dem Lumen des Kanülenrohrs 16 in Verbindung steht und der an seinem Ende mit einem Luer-Lock-Verbinder 21 versehen ist, um während der Punktion eine Flüssigkeit injizieren zu können. Das Kanülenrohr 16 hat eine solche Länge, daß es in den Kurzkatheter 10 eingeschoben werden kann. Wenn der Katheteransatz 14 gegen den Kanülenansatz 19 stößt, ragt nur noch die leitende Spitze 17 aus dem Katheterrohr 12 heraus.

Das aus Stahl bestehende Kanülenrohr 16 ist mit einem Draht verbunden, der als isoliertes Kabel 22 aus dem Kanülenansatz 19 herausgeführt ist. Am Ende des Kabels 22 befindet sich eine elektrische Anschlußvorrichtung in Form eines Steckverbinders 23.

Diese Anschlußvorrichtung 23 wird an das eine Kabel 24 eines Stimulationsgerätes 26 angeschlossen, welches die Stimulationsimpulse in Form elektrischer Spannungsimpulse erzeugt. Das Kabel 24 ist mit einem Steckverbinder 28 versehen, der mit dem Steckverbinder 23 zusammenpaßt. Das andere Kabel 25 des Stimulationsgerätes ist mit einer EKG-Klammer 27 verbunden, die an eine (nicht dargestellte) Hautelektrode angesetzt werden kann, welche nahe der Punktionsstelle befestigt wird.

Bei der Punktion ist zunächst der Steckverbinder 23 der Punktionskanüle 11 mit dem Steckverbinder 28 des Stimulationsgerätes 26 verbunden. Dadurch gelangen die elektrischen Stimulationsimpulse an die blanke Spitze 17 der Stahlkanüle 11. Wenn diese Spitze sich in der Nähe eines Nervenstranges befindet, werden die Nerven zu Reflexbewegungen angeregt. Dadurch ist für den Anästhesisten erkennbar, ob die Nähe des für die Nervenblockade geeigneten Bereichs erreicht wurde. Dabei wird in der Regel die die Nerven umgebende Faszie durchstochen, ohne daß allerdings die Nerven beschädigt werden dürfen.

Im Anschluß an die beschriebene Punktion wird die Stahlkanüle 11 aus dem Kurzkatheter 10 herausgezogen und die Steckverbinder 23 und 28 werden getrennt. Dann wird der Plexuskatheter 30 mit dem darin liegenden Stimulationsdraht 31 durch den Katheteransatz 14 in den Kurzkatheter 10 eingeführt. Der Plexuskatheter 30 ist ein flexibler Schlauch mit einem Außendurchmesser von etwa 1 mm. An diesem Schlauch befinden sich Markierungen, die dem Anästhesisten die Einschubtiefe anzeigen. Durch den Plexuskatheter 30 hindurch verläuft der Stimulationsdraht 31, der hinter der geschlossenen Katheterspitze endet. Am distalen Ende des Plexuskatheters 30 befindet sich eine geschlossene Katheterspitze 32 in Form einer abgerundeten Kuppe. Das distale Ende des Stimulationsdrahtes 31 stößt gegen die Innenwand der Katheterspitze 32. Wie aus Fig. 2 hervorgeht, besteht zwischen dem Stimulationsdraht 31 und der Innenwand des Plexuskatheters 30 ein Ringspalt 33. Ein Plexuskatheter 30, dessen Innendurchmesser 0,7 mm beträgt, enthält einen Stimulationsdraht 31, dessen Durchmesser 0,5 mm beträgt. Der Ringspalt 33 ermöglicht ein leichtes Verschieben des Stimulationsdrahtes 31 im Plexuskatheter 30. Ferner ermöglicht er auch die Injektion eines flüssigen Anästhetikums.

In der Nähe der Katheterspitze 32 sind seitlich am Plexuskatheter 30 Löcher 34 vorgesehen, die durch die Wand des Plexuskatheters hindurchgehen. Die Löcher 34 sind umfangsmäßig äquidistant verteilt und in Längsrichtung des Plexuskatheters gegeneinander versetzt. Im vorliegenden Fall sind drei Löcher 34 vorhanden, die umfangsmäßig um jeweils 120° zueinander versetzt sind. Die Löcher 34 begrenzen jeweils eine Elektrode 35, die aus dem durch das Loch exponierten Teil der blanken Oberfläche des Stimulationsdrahtes 31 besteht. In Fig. 2 sind die elektrischen Stromlinien 36 dargestellt, die von der Elektrode 35 ausgehen und durch das Körpergewebe hindurch verlaufen. Mit 37 ist ein in der Nähe befindlicher Nerv bezeichnet, der von den Stromlinien 36 erfaßt und stimuliert wird.

Das proximale Ende des Stimulationsdrahtes 31, das aus dem Plexus-Katheter 30 herausragt, ist mit einem Steckverbinder 38 versehen, der in gleicher Weise ausgebildet ist, wie der Steckverbinder 23 und der daher mit dem Steckverbinder 28 des Stimulationsgerätes 26 zusammenpaßt.

Nach dem Verlegen des Kurzkatheters 10 und dem Herausziehen der Stahlkanüle 11 wird der Plexuskatheter 30 mit dem darinliegenden Stimulationsdraht 31 in den Kurzkatheter 10 eingeschoben. Dabei sind die Steckverbinder 28 und 38 zusammengsteckt. Unter Stimulation wird nun der Plexuskatheter 30 zusammen mit dem Stimulationsdraht 31 weiter vorgeschoben, so daß unter Stimulationskontrolle diejenige Stelle gefunden werden kann, an der ein an der Katheterspitze 37 injiziertes Medikament die größte Wirksamkeit entfaltet. Nach dem Plazieren des Plexuskatheters 30 wird der Stimulationsdraht 31 herausgezogen und an das proximale Katheterende 39 wird ein Anschlußstück 40 angesetzt, welches einen Luer-Lock-Anschluß 41 zum Ankuppeln einer Spritze, die die zu injizierende Flüssigkeit enthält, aufweist.

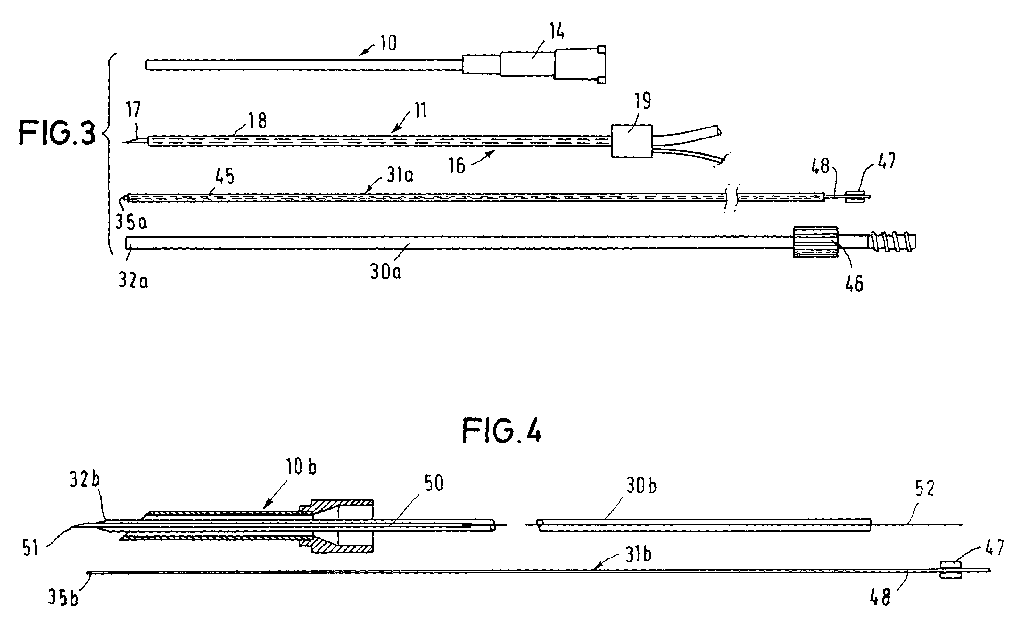

Während bei dem ersten Ausführungsbeispiel der Plexuskatheter 30 zusammen mit dem darinliegenden Stimulationsdraht 31 durch den Kurzkatheter 10 hindurchgeschoben wird, ist in Fig. 3 ein Katheterset dargestellt, bei dem der Plexuskatheter 30a nach der Seldinger-Methode verlegt wird. Hierzu sind ein Kurzkatheter 10 und eine Stahlkanüle 11 vorgesehen, die den entsprechenden Teilen des ersten Ausführungsbeispiels entsprechen. Der Stimulationsdraht 31a ist dicker als der Stimulationsdraht 31 und er ist mit einem Isoliermantel 45 versehen, der nur die Stimulationselektrode 35a und den Anschlußbereich freiläßt.

Der Plexuskatheter 30a gemäß Fig. 3 weist am proximalen Ende einen fest angebrachten Katheteransatz 46 auf und ist am distalen Ende offen.

Bei Benutzung des Kathetersets nach Fig. 3 wird zunächst mit der Stahlkanüle 11 und dem darauf aufgeschoben Kurzkatheter 10 die Punktion durchgeführt, wobei das Einführen unter Stimulationskontrolle erfolgt. Dann wird die Stahlkanüle 11 aus dem Kurzkatheter 10 herausgezogen und der Stimulationsdraht 31a wird in den Kurzkatheter 10 eingeschoben, wobei eine Kontaktklemme 47 an den Anschlußbereich 48 angesteckt ist. Das weitere Vorschieben des Stimulationsdrahtes 31a über die Spitze des Kurzkatheters 10 heraus erfolgt also ebenfalls unter Stimulationskontrolle.

Wenn die Stimulationselektrode 35a die gewünschte Stelle erreicht hat, wird die Kontaktklemme 47 entfernt und vom proximalen Ende des Stimulationsdrahtes 31a her wird der Stimulationskatheter 30a über den Stimulationsdraht 31a geschoben. Am Stimulationsdraht 31a befinden sich Markierungen, die anzeigen, wann die Katheterspitze 32a die Spitze des Stimulationsdrahtes erreicht hat. Danach wird der Stimulationsdraht 31a aus dem Plexuskatheter 30a herausgezogen. An den Katheteransatz 46 kann dann eine Spritze angeschlossen werden.

Das Ausführungsbeispiel von Fig. 4 entspricht im wesentlichen dem Katheterset von DE 44 43 672 C2. Hierbei ist eine Stahlkanüle 10b vorgesehen, mit der eine Punktion vorgenommen wird, während der Plexuskatheter 30b in der Stahlkanüle 10b liegt. Der Plexuskatheter 30b enthält eine Nadel 50 mit scharfer Spitze 51, die aus der Katheterspitze 32b herausragt. Das rückwärtige Ende der Nadel 50 ist mit einem Draht 52 verbunden, der aus dem Plexuskatheter 30b herausragt. Nach dem Einführen des Plexuskatheters 30b mit Hilfe der Nadel 50 wird die Stahlkanüle 10b über den Plexuskatheter hinweg zurückgezogen. Dann wird der Plexuskatheter 30b festgehalten, während die Nadel durch Ziehen an dem Draht 52 aus ihm herausgezogen wird. Anschließend wird ein Stimulationsdraht 31b in den Plexuskatheter 30b eingeschoben, bis die Stimulationselektrode 35b am distalen Ende des Stimulationsdrahtes aus der Katheterspitze 32b hervorsteht. Dann kann der Plexuskatheter 30b zusammen mit dem Stimulationsdraht 31b weiter vorgeschoben werden. Die Stimulationselektrode 35b bildet eine stumpfe Spitze, durch die keine Nerven beschädigt werden können. Während des Vorschiebens ist an den Anschlußbereich 48 eine Kontaktklemme 47 angeschlossen, so daß das Vorschieben unter Stimulationskontrolle durchgeführt werden kann. Wenn der Plexuskatheter 30b seine endgültige Position erreicht hat, wird der Stimulationsdraht 31b aus ihm herausgezogen.

## Patentansprüche

1. Katheterset für die Plexusanästhesie, mit
einer Stahlkanüle (11),
einem auf die Stahlkanüle (11) aufschiebbaren Kurzkatheter (12),
einem einen Stimulationsdraht (31) enthaltenden Plexuskatheter (30), der mit dem innenliegenden Stimulationsdraht (31) durch den Kurzkatheter (10) hindurchschiebbar ist,
wobei der Stimulationsdraht (31) an seinem distalen Ende mindestens eine freiliegende Stimulationselektrode (35) und am proximalen Ende ein elektrisches Anschlußelement (38) aufweist.

2. Katheterset nach Anspruch 1, dadurch gekennzeichnet, daß die Stahlkanüle (11) eine elektrische Anschlußvorrichtung (23) aufweist.

3. Katheterset nach Anspruch 1 oder 2, dadurch gekennzeichnet, daß ein von dem Plexuskatheter (30) lösbares Anschlußstück (40) vorgesehen ist.

4. Katheterset nach einem der Ansprüche 1-3, dadurch gekennzeichnet, daß der Plexuskatheter (30) am distalen Ende (32) geschlossen ist und in der Nähe des distalen Endes mindestens ein seitliches Loch (34) aufweist, welches die Stimulationselektrode (35) freigibt.

5. Katheterset zur Verlegung eines Plexuskatheters für die Plexusanästhesie, mit
einer Stahlkanüle (11),
einem auf die Stahlkanüle (11) aufschiebbaren Kurzkatheter (10),
einem nach Entfernen der Stahlkanüle (11) durch den Kurzkatheter (10) schiebbaren Stimulationsdraht (31a), der an seinem distalen Ende eine Stimulationselektrode (35a) und am proximalen Ende eine elektrische Anschlußmöglichkeit (48) aufweist, und
einem Plexuskatheter (30a), der nach dem Abziehen des Kurzkatheters (10) auf den Stimulationsdraht (31a) aufschiebbar ist.

6. Katheterset nach Anspruch 5, dadurch gekennzeichnet, daß der Stimulationsdraht (31a) zwischen der Stimulationselektrode (32) und dem Anschlußbereich (33) einen Isoliermantel (45) aufweist.

7. Katheterset nach Anspruch 5 oder 6, dadurch gekennzeichnet, daß der Plexuskatheter (30a) einen festen Katheteransatz (46) aufweist.

8. Katheterset zur Verlegung eines Plexuskatheters für die Plexusanästhesie, mit
einer Stahlkanüle (10b),
einem durch die Stahlkanüle (10b) hindurchschiebbaren Plexuskatheter (30b),
einer Nadel (50), die derart in dem Plexuskatheter (30b) sitzt, daß ihre Spitze (51) aus der Katheterspitze (32b) vorsteht, und
einem Stimulationsdraht (31b), der nach dem Herausziehen der Nadel (50) aus dem Plexuskatheter (30b) in den Plexuskatheter einführbar ist und am distalen Ende eine Stimulationselektrode (35b) und am proximalen Ende eine Anschlußmöglichkeit (33) aufweist.
